Europäisches Patentamt

**European Patent Office**

**Office européen des brevets**

(11) Publication number: **0 177 288**
**A2**

## (12) EUROPEAN PATENT APPLICATION

(21) Application number: **85306881.5**

(22) Date of filing: **27.09.85**

(51) Int. Cl.⁴: **A 61 F 2/52**

(30) Priority: **03.10.84 US 657301**

(43) Date of publication of application:
**09.04.86 Bulletin 86/15**

(84) Designated Contracting States:
**AT BE CH DE FR GB IT LI LU NL SE**

(71) Applicant: **Baylor College of Medicine**
**Texas Medical Center 1200 Moursund Avenue**
**Houston Texas 77030(US)**

(72) Inventor: **Gerow, Frank Judson**
**12702 Barryknoll**
**Houston Texas 77024(US)**

(74) Representative: **Wilkinson, Stephen John et al,**
**c/o Stevens, Hewlett & Perkins 5 Quality Court Chancery**
**Lane**
**London WC2A 1HZ(GB)**

(54) Labeled breast prosthesis and methods for detecting and predicting rupture of the prosthesis.

(57) Disclosed is a prosthesis for implantation into human soft tissue which is constructed of silicone gel, saline, or a combination of silicone gel and saline contained within a silicone elastomer envelope which has been labeled with radioopaque markers. Labeling the silicone elastomer envelope according to the present invention makes possible the use of roentgenographic imaging to determine whether the silicone elastomer envelope has ruptured and whether the silicone elastomer envelope is folded persistently in a particular location increasing the probability that the envelope may rupture along the fold line. Also disclosed is a method for using roentgenography to determine whether silicone gel has escaped from the silicone elastomer envelope of a prosthesis by labeling the silicone elastomer envelope with radioopaque materials and a method for determining whether fold-fault failure of the silicone elastomer envelope of implanted prostheses is likely to occur.

Croydon Printing Company Ltd

A LABELED BREAST PROSTHESIS AND
METHODS FOR DETECTING AND
PREDICTING RUPTURE OF THE PROSTHESIS

Background of the Invention

Surgical procedures to alter the appearance of the human female breast comprise a substantial portion of the surgery performed by plastic surgeons. It has been estimated that 16 percent of the income derived by plastic surgeons in the United States results from such surgery. The surgical procedures frequently involve implantation of silicone based prostheses. Silicone implant mammoplasty is indicated in a variety of conditions, including cosmetic augmentation in micromastia, reconstructive augmentation in mammary asymmetry, and total reconstruction after mastectomy. The most commonly used breast implants are constructed of silicone gel contained within a silicone elastomer envelope. Less commonly used implants are constructed of a silicone elastomer envelope to which saline can be added to give the desired size or of a medical grade silicone gel-filled implant within a larger silicone elastomer envelope to which saline can be added. The first such implant of each type was developed approximately 20 years ago by the present applicant working in conjuction with another physician and with persons at Dow Corning Corporation. In the years since development, approximately one million pairs of these prostheses have been implanted; and, currently,

-2-

they are being implanted at a rate of approximately one hundred thousand pairs per year.

Prior to development of the silicone elastomer enveloped, silicone gel prostheses, various agents were instilled directly into breasts to alter their appearance. Among the agents used were paraffin, processed petroleum, and silicone fluid. Usage of these directly instilled agents now is disfavored and largely has been discontinued because their use frequently was associated with local, often intense, inflammatory reactions. Additionally, migration of these instilled agents, particularly silicone fluid, has been associated with more generalized, potentially fatal tissue responses which vary depending upon into which tissue or structure the agent migrated. Human Adjuvant Disease may be a systemic effect of paraffin or silicone fluid instillation and is apparently an autoimmune connective tissue disorder that manifests as mixed connective tissue disease, rheumatoid arthritis, systemic scleroderma, or systemic lupus erythematosus. Onset of Adjuvant Disease has been reported as early as 2 years and as many as 25 years following instillation of breast augmentation agents such as paraffin or silicone fluid. In some cases, remission of the disease has followed removal of the augmentation agents; and in two cases, the disease has proved fatal.

Until recently, Adjuvant Disease had not been associated with implantation of silicone elastomer enveloped, silicone gel prostheses. However, within the last two years, there have been several reported cases where Adjuvant Disease followed implantation of these prostheses. In the only reported patient who developed Adjuvant Disease following prosthetic implantation where the prosthesis has been removed and examined, the silicone elastomer envelope was found to be ruptured; and free silicon gel was found free in the human cavity for the implant. Baldwin, C. M., and Kaplan, E. N., Silicone-Induced Human Adjuvant Disease, 10 Annals of Plastic Surgery 270 (1983). It also is known now that the silicone gel contained in these prostheses is composed of 96 to 98 percent extractable polydimethylsiloxanes (silicone fluid). Thus, the finding of Adjuvant Disease in a patient carrying a ruptured prosthesis combined with data establishing possible association of this disease with instillation of free silicone fluid indicates that the risk of developing Adjuvant Disease may be enhanced by rupture of silicone elastomer enveloped, silicone gel implants. Evidence suggests that patients predisposed to developing Adjuvant Disease are more susceptible to the disease following exposure to a foreign antigen.

Additionally, escape of the silicone gel from the envelope of the prosthesis is associated with other adverse consequences including satelliting of the gel into various parts of the soft tissue around the prosthetic pocket or down into the arm. Silicone gel in the soft tissue forms dense capsules and causes pain necessitating surgical excision of the gel. Recent data also suggest that the risk of infection in the tissue surrounding the prosthesis is increased following its rupture; and free silicone gel in the prosthetic pocket together with an infection may enhance synergistically the host tissue reactions to the implant. Therefore, ruptured prostheses should be removed as soon as possible; and if there is any evidence of infection or movement of the silicone gel away from the prosthetic cavity, ruptured prostheses should be removed immediately.

There are a number of causes of rupture of silicone elastomer enveloped, silicone gel prostheses. The prostheses can be punctured or torn accidently during implantation procedures. If, after implantation, fluid which must be removed accumulates in the prosthetic pocket, a needle inserted to aspirate the fluid may pierce the silicone elastomer envelope. In 10 to 30 percent of patients receiving a prosthetic implant, a

-4-

tight periprosthetic capsule forms which must be released. A closed capsulotomy, during which the breast is compressed forcefully by the surgeon's hands, is a procedure used to break the capsule and soften the feel of the breast. In approximately 30 percent of patients, a closed capsulotomy causes rupture of the prosthetic silicone elastomer envelope. Additionally, rupture of the silicone elastomer envelope can result from biodegradation of the envelope by tissue enzymes. Because of the relatively poor tear resistance of the silicone elastomer envelope, once a rupture or tear has occurred, it will propagate upon only minimal manipulation of the breast, freeing the silicone gel to escape into the prosthetic cavity and beyond.

Fold-fault failure also has been shown to be a cause of rupture of silicone elastomer envelopes, especially in prostheses containing saline. After implantation into the prosthetic cavity, the prosthetic envelope can become folded persistently along a particular line. By abrasion against itself along the fold line, the envelope progressively will wear away causing it to rupture along the fold line. Fold-fault failure of saline containing prostheses creates an emergency because the saline which leaks from the ruptured envelope is absorbed into the surrounding tissues. Absorption of the saline causes the prosthetic cavity to shrink. Thus, to avoid the need for a complete reoperation, rather than mere replacement of the prosthesis, the ruptured prosthesis must be removed immediately after rupture and replaced.

Although silicone elastomer enveloped silicone gel breast prostheses have been used extensively for 20 years and despite a demonstrated need to determine if silicone gel has escaped its envelope, no prosthesis or non-invasive method has been developed which enables determination of whether implanted prostheses have ruptured prior to the onset of complication-related signs and symptoms. Also, no method has been developed to predict an impending fold-fault failure. In most cases, surgeons are unable to determine by external physical examination whether a prosthesis remains intact. Roentgenographic examination also has proved incapable of discerning the status of prosthetic envelopes because, although different from the density of the surrounding soft tissue, the X-ray density of the envelope and the silicone gel are the same. Therefore, roentgenography enables determination of whether the silicone gel remains in the prosthetic cavity but provides no indication of whether the silicone gel is contained within the envelope or has escaped into the cavity thus freeing the gel to migrate into surrounding or distant soft tissues and provides no way of predicting an impending fold-fault failure.

This long-felt need for a prosthesis and a procedure which enables determination of whether implanted silicone elastomer enveloped, silicone gel implants remain intact now has been satisfied. The present invention exploits the tendency of the silicone gel to adhere to the external surface of the silicone elastomer envelope after escaping the confines of the envelope. Thus, after the silicone elastomer envelope breaks, a large portion of the silicone gel that is free in the prosthetic cavity remains adhered to the external surface of the silicone elastomer envelope forcing the envelope away from the surrounding tissue. Implantation of a prosthesis composed of a silicone elastomer envelope marked with a material which absorbs electromagnetic energy to an extent different from silicone gel and different from the surrounding tissue enables production of an image, by irradiating the tissue bearing a marked implant with electromagnetic energy, which shows the location of the silicone gel relative to the silicone elastomer envelope. An image showing no silicone gel between the surrounding tissue and the silicone elastomer envelope establishes that the silicone gel remains within the envelope. Conversely, when the image produced shows silicone gel interposed between the surrounding tissue and the silicone elastomer envelope, it is clear that the envelope has ruptured.

The material that absorbs electromagnetic energy to an extent different from silicone gel and different human soft tissue used to mark the silicone elastomer envelope can be incorporated within the envelope during manufacture or can be applied to the envelope following manufacture of the prosthesis. Marking the silicone elastomer envelope with one of several readily available radioopaque materials and producing an image by X-irradiation of the breast, as is done in mammography, has proved a successful application of this invention. However, the present invention equally is applicable to evaluating the prosthetic envelope status by examining images produced by irradiating breasts bearing appropriately marked prostheses with energy from other segments of the electromagnetic spectrum. The present innovation also is applicable to determining the status of other types of silicone elastomer enveloped, silicone gel prostheses and other types of silicone elastomer prostheses. Such other types of prostheses include various facial implants, penile implants, testicular implants, and carpal, metacarpal, and interphalangeal joint replacements and similar joint replacements used in the feet.

Further, marking the silicone elastomer envelope of saline containing prostheses provides a method for predicting fold-fault failure. To predict an impending failure, two or more serial electromagnetic energy produced images, such as mammograms, are compared. If the same fold line persists, the probability that rupture along that fold line will occur is enhanced.

Also, a mammogram obtained while the breast was compressed along its lateral and medial aspects can be compared to one obtained absent breast compression. If the same fold line appears in the compressed and non-compressed mammograms, the probability that the identified fold line is persistent and will be a point of rupture is increased. Once a persistent fold line is detected, the surgeon can employ external manipulation or other techniques to alleviate the persistent fold, thus preventing rupture of the prosthetic envelope.

Prior Art Statement

Applicant is unaware of any prior art teaching marking the silicone elastomer envelope of silicone gel containing prostheses with materials that absorb electromagnetic energy to an extent different from silicone gel and different from breast tissue, any prior art which teaches using electromagnetic energy to produce images of breasts bearing implants so marked to ascertain whether the silicone elastomer envelopes of the prostheses have ruptured or any prior art which teaches using electromagnetic energy to produce images of breasts so marked to enable prediction of fold-fault failure of prosthetic silicone elastomer envelopes.

Prior art does teach the use of radioopaque markers to enable roentgenographic determination of the location of various types of medical devices. For example, catheters placed in the subclavian veins for administration of intravenous hyperalimentation bear X-ray detectable markers so that proper positioning of the catheter can be confirmed prior to initiation of administration of the hyperalimentation fluid. Similar position-indicating markers also are used on chest tubes, cardiac catheters, surgical sponges, and other intravascular devices. These markers enable roentgenographic determination of the course and location of the device; but no information about the volume or configuration of the device and no information about whether the device remains volumetrically intact can be gained using position-indicating markers.

The prior art also teaches using radioopaque agents such as barium sulfate to determine the configuration and patency of the lumen of the gastrointestinal tract. Although the silicone gel contained within the envelope of a silicone-based prosthesis could be marked with barium sulfate, such marking would fail to provide any way of determining whether the marked gel was within or without the silicone elastomer envelope.

## Summary of the Invention

The present invention is directed to breast prostheses composed of silicone gel contained within a silicone elastomer envelope labeled with a radioopaque marker which enables roentgenographic determination of whether the envelope has ruptured, to a method for using roentgenographic imaging to determine whether silicone elastomer envelopes of silicone gel containing prostheses have ruptured and to a method for using roentgenographic imaging to predict impending fold-fault failure of prosthetic silicone elastomer envelopes.

Accordingly, it is an object of the present invention to provide a breast prosthesis composed of silicone gel or silicone gel and saline contained in a silicone elastomer envelope which has been labeled with a radioopaque marker to enable roentgenographic determination of whether the envelope has ruptured.

A further object of the present invention is to provide a method for using roentgenographic imaging to determine whether the silicone elastomer envelope of a silicone gel containing prosthesis has ruptured.

A still further object of the present invention is to provide a method for using roentgenographic imaging

-9-

to predict fold-fault failure of prosthetic silicone elastomer envelopes.

Other and further objects, features, and advantages appear throughout.

Brief Description of the Drawings

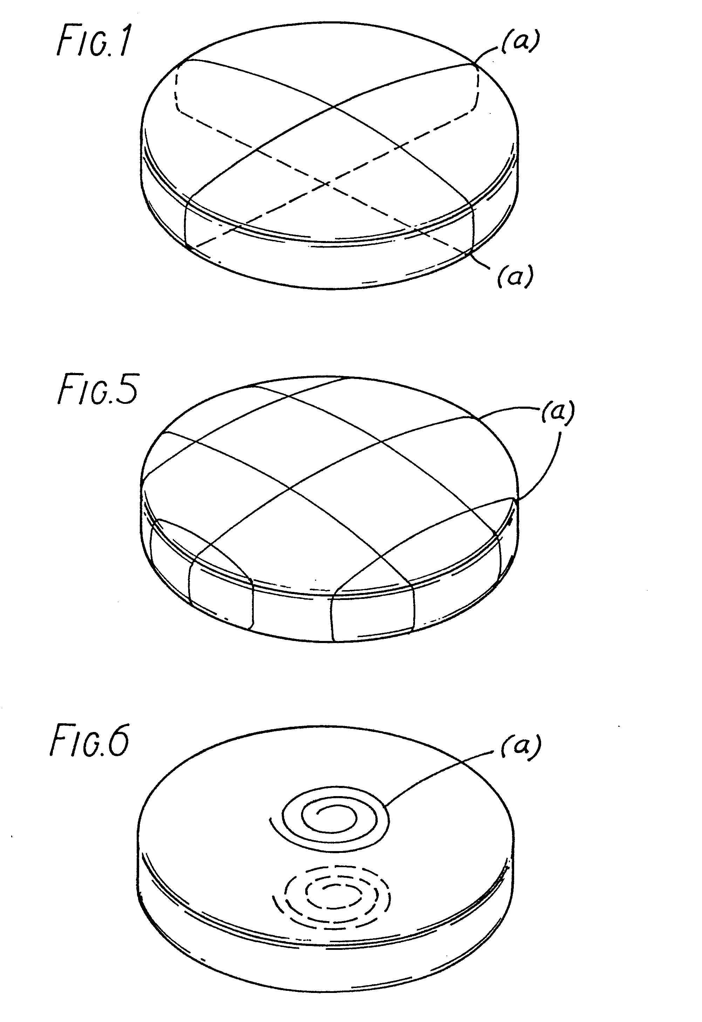

Figure 1 is an elevational view of a spheroid prosthesis resting on a horizontal flat surface wherein the prosthesis is constructed of silicone gel, saline, or silicone gel and saline contained within a silicone elastomer envelope to which two strips of radioopaque material have been affixed so that one strip encircles the prosthesis in the direction if its X axis and the other strip encircles the prosthesis in the direction of its Y axis.

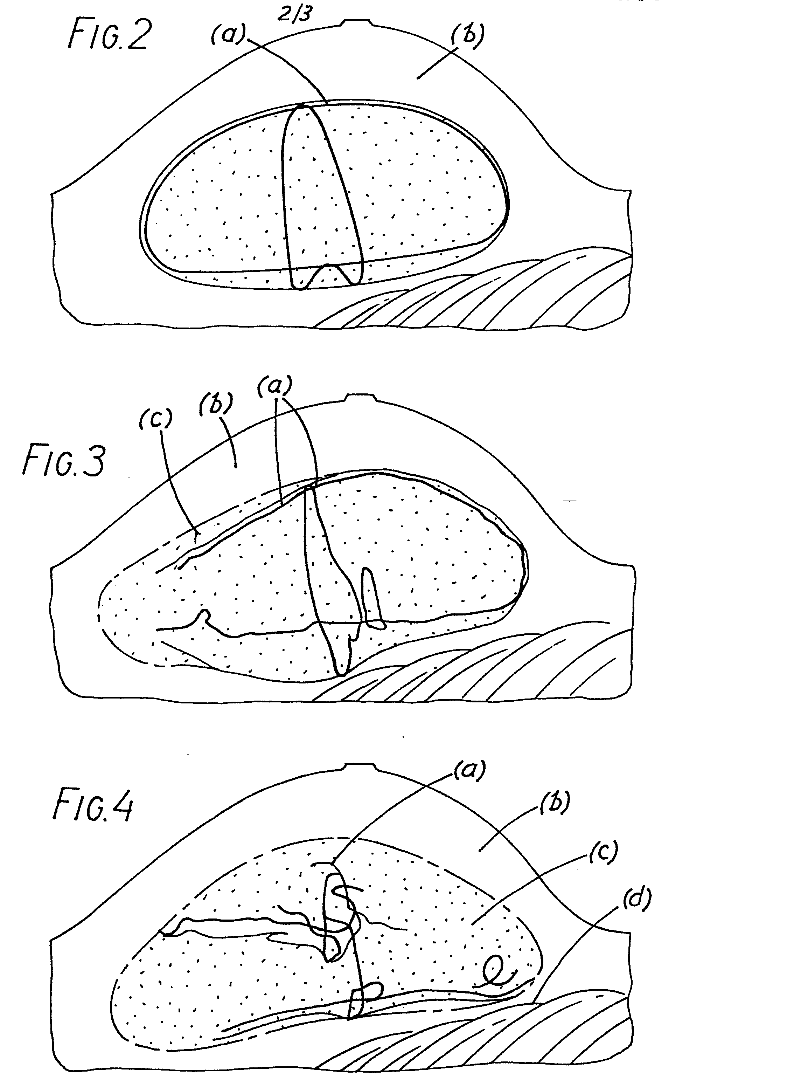

Figure 2 is a mammogram of a breast bearing a prosthesis marked as in Figure 1 wherein the silicone elastomer envelope of the prosthesis is intact.

Figure 3 is a mammogram of a breast bearing a prosthesis marked as in Figure 1 wherein the silicone elastomer envelope of the prosthesis has ruptured, and some of the silicone gel has escaped from the silicone elastomer envelope.

Figure 4 is a mammogram of a breast bearing a prosthesis marked as in Figure 1 wherein the silicone elastomer envelope of the prosthesis has ruptured, and most of the silicone gel has escaped from the silicone elastomer envelope.

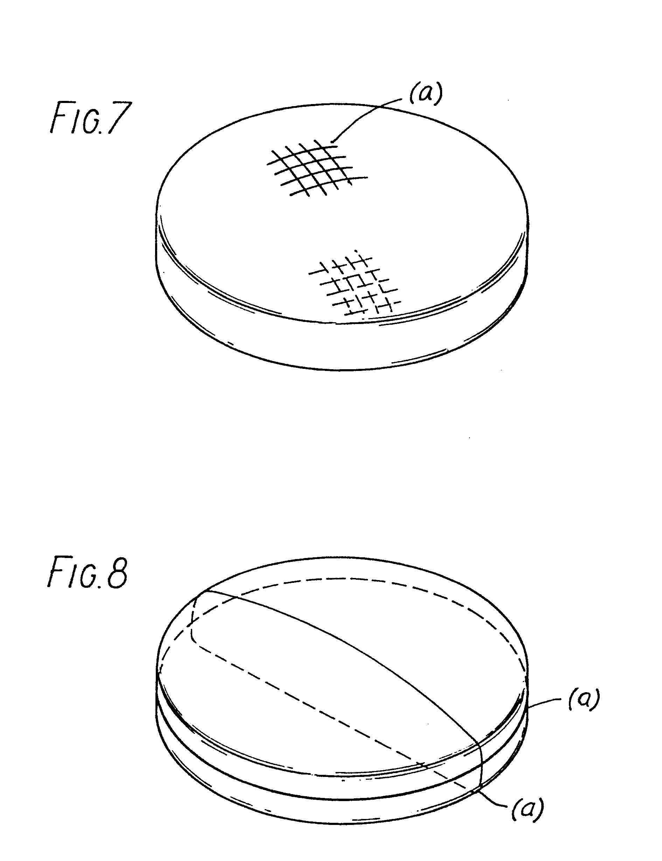

Figure 5 is an elevational view of a spheroid prosthesis resting on a horizontal flat surface wherein the prosthesis is constructed of silicone gel, saline, or silicone gel and saline contained within a silicone elastomer envelope to which strips of radioopaque material have been affixed so that the strips intersect to form a mesh-like pattern over the entire envelope.

-10-

Figure 6 is an elevational view of a spheroid prosthesis resting on a horizontal flat surface wherein the prosthesis is constructed of silicone gel, saline, or silicone gel and saline contained within a silicone elastomer envelope to which two strips of radioopaque material have been affixed so that one strip is in a coiled configuration on the top surface of the silicone elastomer envelope, and the second strip is in a coiled configuration on the bottom surface of the silicone elastomer envelope.

Figure 7 is an elevational view of a spheroid prosthesis resting on a horizontal flat surface wherein the prosthesis is constructed of silicone gel, saline, or silicone gel and saline contained with a silicone elastomer envelope to which patches of strips of radioopaque material in mesh-like configurations have been affixed.

Figure 8 is an elevational view of a spheroid prosthesis resting on a horizontal flat surface wherein the prosthesis is constructed of silicone gel, saline, or silicone gel and saline contained within a silicone elastomer envelope to which two strips of radioopaque material have been attached so that one strip encircles the prosthesis in its vertical plane and the other strip encircles the prosthesis in its horizontal plane.

Description of the Preferred Embodiments

Example 1

Although the present applicant recognizes that it is commercially preferable to incorporate the radioopaque material into the silicone elastomer envelope of a prosthesis during its manufacture, in this and the subsequent examples the radioopaque material was applied to the envelopes of previously manufactured prostheses. In this embodiment of the present invention, a mammary prosthesis constructed of silicone gel contained within a soft, seamless silicone elastomer envelope was labeled with two narrow, elastic strips of radioopaque material.

The prosthesis selected is marketed by Wright Dow Corning, Inc., under its registered "Silastic" trademark. The strips of radioopaque material were taken from roentgenographically-detectable surgical sponges sold by Johnson & Johnson Products, Inc., under its registered "Ray-Tec" trademark. The radioopaque strips were attached to the prosthesis by positioning the strips on the prosthetic envelope, completely covering the strips with a small amount of silicone rubber, and recuring the prosthesis by heating to affix the radioopaque label to the envelope permanently.

Figure 1 illustrates one presently preferred configuration for applying the radioopaque label strips (a) to the external surface of the silicone elastomer envelope of a spheroid prosthesis. In this example, the prosthesis is resting on a horizontal flat surface and one label strip has been applied so that it encircles the prosthesis in the direction of its X axis, and another label strip is applied so that it encircles the prosthesis in the direction of its Y axis. Label strips thus applied intersect on the top and bottom surfaces of the prosthesis when viewed from above.

Figure 2 shows a mammogram of a prosthesis marked as above which has been implanted in a breast. This figure illustrates the labeled silicone elastomer envelope (a) in complete contact with the surrounding breast tissue (b) establishing that the envelope remains intact.

In contrast, Figure 3 shows a mammogram of a breast bearing a labeled prosthesis in which the prosthetic envelope (a) has ruptured and a small portion of the silicone gel (c) has escaped the envelope. Rupture of the silicone elastomer envelope is established by the presence of material corresponding to the X-ray density of silicone gel (c) interposed between the breast tissue (b) and the labeled silicone elastomer envelope (a).

Figure 4 shows a mammogram of a breast bearing a labeled prosthesis in which the prosthetic envelope (a) has ruptured, and most of the silicone gel (c) has moved out of the envelope. In this figure, so large a quantity of free silicone gel (c) has become interposed between the breast tissue (b) and the silicone elastomer envelope (a) that the labeled envelope (a) has been pushed back against the chest wall (d).

Therefore, because labeled silicone elastomer envelopes of silicone gel containing prostheses characteristically are forced away from the surrounding tissues by silicone gel outside of the envelope, mammography of breasts bearing prostheses labeled as described above provides a reliable method for determining whether the prosthetic silicone elastomer envelope has ruptured.

Application of radioopaque label strips as described in this example also makes possible predicting whether fold-fault failure of the silicone elastomer envelope of implanted prostheses is impending. The mammogram shown in Figure 2 shows two locations of folding (c) and (d) of the prosthetic envelope. Folding in these same locations of the prosthetic envelope appearing on subsequent mammograms indicates that the folds are persistant and suggests that rupture of the envelope along the fold may occur. Another technique for determining whether a fold is persistent is to compare a mammogram of the breast taken without compressing the breast to the mammogram shown in Figure 2 which was taken with breast compression along the lateral and medial aspects of the breast. The appearance of a fold in the same location of a mammogram obtained with breast compression and one obtained without breast compression also indicates persistence of the fold. Once a persistent fold in the prosthetic envelope has been detected, procedures to alleviate the fold can be initiated to

-13-

prevent rupture of the envelope, thus preventing the possiblity of having to replace the prosthesis on an emergency basis.

### Example 2

In this embodiment of the present invention, the same type of breast prosthesis, the same type of radioopaque labeling material, and the same method for attaching the radioopaque material to the silicone elastomer envelope of the prosthesis, as were used in Example 1, were employed. However, in this example, the radioopaque strips were attached in another presently preferred configuration which differs from that described previously. As shown in Figure 8, two strips of radioopaque material have been attached to the silicone elastomer envelope so that one strip encircles the prosthesis in its vertical plane, and the other strip encircles the prosthesis in its horizontal plane.

Mammography of breasts bearing prostheses labeled as described in this example also was used to evaluate the status of the prosthesis. As long as the silicone elastomer envelope of the prosthesis remained intact, no silicone gel was found between the breast tissue and the labeled prosthetic envelope. However, once the envelope had ruptured, the labeled silicone elastomer envelope was forced away from the surrounding breast tissue; and silicone gel which had migrated from the confines of the envelope was found interposed between the breast tissue and the envelope. Application of radioopaque label strips as described in this example also makes possible use of the method described in Example 1 to predict whether fold-fault failure of the silicone elastomer envelope of implanted prostheses is impending.

### Example 3

In this embodiment of the present invention, the same type of breast prosthesis, the same type of radioopaque labeling material, and the same method for attaching the radioopaque material to the silicone elastomer envelope of the prosthesis, as were used in Example 1, were employed. However, in this example, the radioopaque strips were attached in another presently preferred configuration which differs from that described in the previous example. As shown in Figure 6, viewing the spheroid prosthesis from above, one strip of radioopaque material (a) was affixed to the top external surface of the silicone elastomer envelope in a coiled configuration; and a second such strip was affixed to the bottom external surface of the silicone elastomer envelope in a coiled configuration.

Mammography of breasts bearing prostheses labeled as described in this example also was used to evaluate the status of the prosthesis. As long as the silicone elastomer envelope of a prosthesis remained intact, no silicone gel was found between the breast tissue and the labeled prosthetic envelope. However, once the envelope had ruptured, the labeled silicone elastomer envelope was forced away from the surrounding breast tissue; and silicone gel which had migrated from the confines of the envelope was found interposed between the breast tissue and the envelope.

### Example 4

In this embodiment of the present invention, the same type of breast prosthesis, the same type of radioopaque labeling material, and the same method for attaching the radioopaque material to the silicone elastomer envelope of the prosthesis, as were used in Example 1, were employed. However, in this example, the radioopaque strips were attached in a presently preferred configuration which differs from those described in the previous examples. As shown in Figure 5, several strips of radioopaque material (a) were affixed to the external surface of the silicone elastomer envelope in a mesh-like configuration encasing the entire envelope. The size of the openings in the mesh shown in Figure 5 is merely one possibility; mesh openings much smaller than or larger than those shown in Figure 5 would perform equally well.

Mammography of breasts bearing the prosthesis labeled as described in this example also was used to evaluate the status of the prosthesis. As long as the silicone elastomer envelope of the prosthesis remained intact, no silicone gel was found between the breast tissue and the labeled prosthetic envelope. However, once the envelope had ruptured, the labeled silicone elastomer envelope was forced away from the surrounding breast tissue; and silicone gel which had migrated from the confines of the envelope was found interposed between the breast tissue and the envelope. Application of radioopaque label strips as described in this example also makes possible use of the method described in Example 1 to predict whether fold-fault failure of the silicone elastomer envelope of implanted prostheses is impending.

### Example 5

In this embodiment of the present invention, the same type of breast prosthesis, the same type of radioopaque labeling material, and the same method for attaching the radioopaque material to the silicone elastomer envelope of the prosthesis, as were used in Example 1, were employed. However, in this example, the radioopaque strips were attached in another presently preferred configuration which differs from those previously described. As shown in Figure 7, patches of strips of radioopaque material in a mesh-like configuration (a) were applied to the external surface of the silicone elastomer envelope. As in the previous examples, mammography of breasts bearing prostheses as described in

-16-

this example was used to determine whether the silicone elastomer envelope of an implanted prosthesis remained intact.

## Example 6

In this embodiment of the present invention, the same type of breast prosthesis as was used in Example 1 was employed. However, in this example, the entire surface of the silicone elastomer envelope of the prosthesis was labeled with a radioopaque material. Barium sulfate was selected as the radioopaque material. A quantity of barium sulfate sufficient to produce the desired radio-opacity was mixed with silicone rubber which was then applied in a thin layer to the silicone elastomer envelope of a prosthesis. The prosthesis was then recured by heating to affix the silicone rubber bearing the radioopaque label to the envelope permanently. Breasts bearing prostheses labeled as described in this example were evaluated by mammography to ascertain whether the prosthetic envelope remained intact. As previously described, a mammogram showing silicone gel interposed between the breast tissue and the labeled silicone elastomer envelope established that the enveloped had ruptured.

The present invention, therefore, is well suited and adapted to attain the objects and ends and has the advantages mentioned as well as others inherent therein.

WHAT IS CLAIMED IS:

0177288

-1-

## Claims

1. A prosthesis which allows for determining by electromagnetic energy imaging whether it has ruptured after implantation, comprising:

(a) an inner material that absorbs electromagnetic energy to an extent different from human soft tissue; and

(b) an outer material that absorbs electromagnetic energy to an extent different from the inner material and different from human soft tissue enveloping the inner material.

2. A prosthesis labeled to allow for determining whether it has ruptured after implantation by electromagnetic energy imaging, comprising:

(a) an inner material that absorbs electromagnetic energy to an extent different from human soft tissue;

(b) an outer material that absorbs electromagnetic energy to an extent indistinguishable by electromagnetic energy imaging from the inner material enveloping the inner material; and

(c) a labeling material that absorbs electromagnetic energy to an extent different from the inner material and different from human soft tissue affixed to or incorporated within the outer material.

3. The prosthesis of Claim 2 wherein the inner material is selected from the group consisting of silicone gel, saline, and a combination of silicone gel and saline.

4. The prosthesis of Claim 2 wherein the outer material comprises a silicone elastomer envelope.

5. The prosthesis of Claim 3 wherein the outer material comprises a silicone elastomer envelope.

6. The prosthesis of Claim 2 wherein the labeling material comprises a radioopaque material.

7. The prosthesis of Claim 4 wherein the labeling material comprises a radioopaque material.

8. The prosthesis of Claim 5 wherein the labeling material comprises a radioopaque material.

9. The prosthesis of Claim 6 wherein the radioopaque material comprises two strips affixed to the outer material so that, when the prosthesis is resting on a horizontal flat surface, the first strip encircles the outer material in the direction of the X axis of the prosthesis, and the second strip encircles the outer material in the direction of the Y axis of the prosthesis.

10. The prosthesis of Claim 7 wherein the radioopaque material comprises two strips affixed to the silicone elastomer envelope so that, when the prosthesis is resting on a horizontal flat surface, the first strip encircles the silicone elastomer envelope in the direction of the X axis of the prosthesis, and the second strip encircles the silicone elastomer envelope in the direction of the Y axis of the prosthesis.

11. The prosthesis of Claim 8 wherein the radioopaque material comprises two strips affixed to the silicone elastomer envelope so that, when the prosthesis is resting on a horizontal flat surface, the first strip encircles the silicone elastomer envelope in the direction of the X axis of the prosthesis, and the second strip encircles the silicone elastomer envelope in the direction of the Y axis of the prosthesis.

12. The prosthesis of Claim 6 wherein the radioopaque material comprises two strips affixed to the outer material so that, when the prosthesis is resting on a horizontal flat surface, the first strip encircles the outer material in its vertical plane and the second strip encircles the outer material in its horizontal plane.

13. The prosthesis of Claim 7 wherein the radioopaque material comprises two strips affixed to the silicone elastomer envelope so that, when the prosthesis is resting on a horizontal flat surface, the first strip encircles the silicone elastomer envelope in its horizontal plane and the second strip encircles the silicone elastomer envelope in its vertical plane.

14. The prosthesis of Claim 8 wherein the radioopaque material comprises two strips affixed to the silicone elastomer envelope so that, when the prosthesis is resting on a horizontal flat surface, the first strip encircles the silicone elastomer envelope in its horizontal plane and the second strip encircles the silicone elastomer envelope in its vertical plane.

15. The prosthesis of Claim 6 wherein the radioopaque material comprises two strips affixed to the outer material so that, when viewed from above, the first strip is in a coiled configuration on the top surface of the outer material, and the second strip is in a coiled configuration on the bottom surface of the outer material.

16. The prosthesis of Claim 7 wherein the radioopaque material comprises two strips affixed to the silicone elastomer envelope so that, when viewed from

-4-

above, the first strip is in a coiled configuration on the top surface of the outer material, and the second strip is in a coiled configuration on the bottom surface of the outer material.

17. The prosthesis of Claim 8 wherein the radioopaque material comprises two strips affixed to the silicone elastomer envelope so that, when viewed from above, the first strip is in a coiled configuration on the top surface of the outer material, and the second strip is in a coiled configuration on the bottom surface of the outer material.

18. The prosthesis of Claim 6 wherein the radioopaque material comprises strips affixed to the outer material so that the strips intersect to form a mesh-like pattern encasing all of the outer material.

19. The prosthesis of Claim 7 wherein the radioopaque material comprises strips affixed to the silicone elastomer envelope so that the strips intersect to form a mesh-like pattern encasing the entire envelope.

20. The prosthesis of Claim 8 wherein the radioopaque material comprises strips affixed to the silicone elastcmer envelope so that the strips intersect to form a mesh-like pattern encasing the entire envelope.

21. The prosthesis of Claim 6 wherein the radioopaque material comprises strips affixed to the outer material so that the strips form patches of mesh-like configurations.

22. The prosthesis of Claim 7 wherein the radioopaque material comprises strips affixed to the silicone elastomer envelope so that the strips form patches of mesh-like configurations.

23. The prosthesis of Claim 8 wherein the radioopaque material comprises strips affixed to the silicone elastomer envelope so that the strips form patches of mesh-like configurations.

24. The prosthesis of Claim 6 wherein the radioopaque material is affixed to the outer material so that the radioopaque material covers the entire surface of the outer material.

25. The prosthesis of Claim 7 wherein the radioopaque material is affixed to the silicone elastomer envelope so that the radioopaque material covers the entire surface of the silicone elastomer envelope.

26. The prosthesis of Claim 8 wherein the radioopaque material is affixed to the silicone elastomer envelope so that the radioopaque material covers the entire surface of the silicone elastomer envelope.

27. A method for determining whether a prosthesis implanted in human soft tissue comprised of an inner material that absorbs electromagnetic energy to an extent different from human soft tissue and an outer material that absorbs electromagnetic energy to an extent indistinguishable by electromagnetic energy imaging from the inner material has ruptured, comprising:
   (a) labeling the outer material of a prosthesis to be implanted with a material which absorbs electromagnetic energy to an extent different from the inner material and different from human soft tissue;

-6-

(b) producing an image showing the implanted prosthesis by irradiating the human soft tissue into which the prosthesis was implanted with electromagnetic energy; and

(c) examining the image to determine if any of the inner material is interposed between the labeled outer material of the prosthesis and the human soft tissue into which the prosthesis was implanted.

28. The method of Claim 27 wherein the material used to label the outer material of the prosthesis is radioopaque.

29. The method of Claim 28 wherein the image is produced by irradiating the human soft tissue into which the prosthesis was implanted with X-irradiation.

30. The method of Claim 29 wherein the radioopaque label is affixed to the outer material in two strips so that, when the prosthesis is resting on a horizontal flat surface, the first strip encircles the outer material in the direction of the X axis of the prosthesis, and the second strip encircles the outer material in the direction of the Y axis of the prosthesis.

31. The method of Claim 29 wherein the radioopaque label is affixed to the outer material in two strips so that, when the prosthesis is resting on a horizontal flat surface, the first strip encircles the outer material in its horizontal plane and the second strip encircles the outer material in its vertical plane.

32. The method of Claim 29 wherein the radioopaque material is affixed to the outer material in two strips so that, when viewed from above, the first strip is in a coiled configuration on the top surface of the outer material, and the second strip is in a coiled configuration on the bottom surface of the outer material.

33. The method of Claim 29 wherein the radioopaque material is affixed to the outer material in strips so that the strips intersect to form a mesh-like pattern over the entire surface of the outer material.

34. The method of Claim 29 wherein the radioopaque material is affixed to the outer material in patches of strips in mesh-like configurations.

35. The method of Claim 29 wherein the radioopaque material is affixed to the outer material so that the radioopaque material completely covers the surface of the outer material.

36. A method for determining whether a silicone elastomer enveloped, silicone gel prosthesis remains intact after implantation into human soft tissue, comprising:
    (a)  labeling the silicone elastomer envelope with a radioopaque material;
    (b)  obtaining an X-ray image of the implanted prosthesis and surrounding soft tissue; and
    (c)  examining the X-ray image to determine if any of the silicone gel has become interposed between the silicone elastomer envelope and the soft tissue.

37. The method of Claim 36 wherein the radioopaque material used to label the silicone elastomer envelope is in two strips affixed to the silicone elastomer envelope so that, when the prosthesis is resting on a horizontal flat surface, the first strip encircles the silicone elastomer envelope in the direction of the X

-8-

axis of the prosthesis, and the second strip encircles the silicone elastomer envelope in the direction of the Y axis of the prosthesis.

38. The method of Claim 36 wherein the radioopaque material used to label the silicone elastomer envelope is in two strips affixed to the silicone elastomer envelope so that, when the prosthesis is resting on a horizontal flat surface, the first strip encircles the silicone elastomer envelope in its horizontal plane and the second strip encircles the silicone elastomer envelope in its vertical plane.

39. The method of Claim 36 wherein the radioopaque material used to label the silicone elastomer envelope is in two strips affixed to the silicone elastomer envelope so that, when viewed from above, the first strip is in a coiled configuration on the top surface of the silicone elastomer envelope and the second strip is in a coiled configuration on the bottom surface of the silicone elastomer envelope.

40. The method of Claim 36 wherein the radioopaque material used to label the silicone elastomer envelope is in strips affixed to the silicone elastomer envelope so that the strips intersect to form a mesh-like pattern over the entire envelope.

41. The method of Claim 36 wherein the radioopaque material used to label the silicone elastomer envelope is in strips affixed to the silicone elastomer envelope so that the strips form patches of radioopaque material in mesh-like configurations.

42. The method of Claim 36 wherein the radioopaque material used to label the silicone elastomer envelope is affixed to the silicone elastomer envelope so that the radioopaque material completely covers the surface of the silicone elastomer envelope.

43. A method for using roentgenography to predict whether fold-fault failure of an implanted silicone elastomer enveloped prosthesis containing silicone gel, saline, or silicone gel and saline is likely to occur, comprising:

(a) labeling the prosthetic silicone elastomer envelope with a radioopaque material prior to implantation of the prosthesis;

(b) obtaining two or more serial roentgenograms of the prosthesis after implantation; and

(c) comparing the roentgenograms to determine if there is a persistent fold line in the silicone elastomer envelope.

44. The method of Claim 43 wherein the radioopaque material used to label the silicone elastomer envelope is affixed to the envelope in two strips so that, when the prosthesis is resting on a horizontal flat surface, the first strip encircles the envelope in the direction of the X axis of the prosthesis, and the second strip encircles the envelope in the direction of the Y axis of the prosthesis.

45. The method of Claim 43 wherein the radioopaque material used to label the silicone elastomer envelope is affixed to the envelope in two strips so that, when the prosthesis is resting on a horizontal flat surface, the first strip encircles the envelope in its horizontal plane and the second strip encircles the envelope in its vertical plane.

46. The method of Claim 43 wherein the radioopaque material used to label the silicone elastomer envelope has been affixed to the envelope in strips so that the strips intersect to form a mesh-like pattern over the entire envelope.

47. A method for using mammography to predict whether fold-fault failure of an implanted silicone elastomer enveloped prosthesis containing silicone gel, saline, or silicone gel and saline is likely to occur, comprising:

(a) labeling the prosthetic silicone elastomer envelope with a radioopaque material prior to implantation of the prosthesis;

(b) obtaining a mammogram of a breast bearing the prosthesis wherein the breast is compressed along its lateral and medial aspects;

(c) obtaining another mammogram of the breast bearing the labeled prosthesis wherein the breast is not compressed; and

(d) comparing the roentgenograms to determine if there is a persistent fold line in the silicone elastomer envelope.

48. The method of Claim 47 wherein the radioopaque material used to label the silicone elastomer envelope is affixed to the envelope in two strips so that, when the prosthesis is resting on a horizontal flat surface, the first strip encircles the envelope in the direction of the X axis of the prosthesis and the second strip encircles the envelope in the direction of the Y axis of the prosthesis.

49. The method of Claim 47 wherein the radioopaque material used to label the silicone elastomer envelope is affixed to the envelope in two strips so that, when the prosthesis is resting on a horizontal flat surface, the first strip encircles the envelope in its horizontal plane and the second strip encircles the envelope in its vertical plane.

50. The method of Claim 47 wherein the radioopaque material used to label the silicone elastomer envelope is affixed to the envelope in strips so that the strips intersect to form a mesh-like pattern over the entire envelope.

FIG.1

(a)

(a)

FIG.5

(a)

FIG.6

(a)

0177288

FIG.2 (a) (b)

FIG.3 (c) (b) (a)

FIG.4 (a) (b) (c) (d)

*FIG.7*

*FIG.8*